(19) Europäisches Patentamt
European Patent Office
Office européen des brevets

(11) Veröffentlichungsnummer: **0 194 469**
**B1**

(12) # EUROPÄISCHE PATENTSCHRIFT

(45) Veröffentlichungstag der Patentschrift: **04.01.89**

(21) Anmeldenummer: **86101988.3**

(22) Anmeldetag: **17.02.86**

(51) Int. Cl.⁴: **C 07 C 143/80,** C 07 D 271/02, C 07 D 455/02, C 09 B 57/00, G 03 G 5/09, H 05 K 1/03

(54) **Neue sulfonhaltige Styrolderivate, Verfahren zu deren Herstellung und deren Verwendung.**

(30) Priorität: **23.02.85 DE 3506436**

(43) Veröffentlichungstag der Anmeldung: **17.09.86 Patentblatt 86/38**

(45) Bekanntmachung des Hinweises auf die Patenterteilung: **04.01.89 Patentblatt 89/1**

(84) Benannte Vertragsstaaten: **DE FR GB NL**

(56) Entgegenhaltungen:
**DE-A-1 497 118**
**DE-A-2 556 620**
**FR-A-2 397 659**

(73) Patentinhaber: **HOECHST AKTIENGESELLSCHAFT, Postfach 80 03 20, D-6230 Frankfurt am Main 80 (DE)**

(72) Erfinder: **Bender, Albert, Dr. Dipl.- Chem., Henry-Moisand- Strasse 16, D-6500 Mainz- Laubenheim (DE)**
Erfinder: **Günther, Dieter, Dr. Dipl.- Chem., Nachtigallenweg 1a, D-6233 Kelkheim (DE)**
Erfinder: **Lingnau, Jürgen, Dr. Dipl.- Chem., Karolinger Strasse 10, D-6500 Mainz- Laubenheim (DE)**

**EP 0 194 469 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung bezieht sich auf neue sulfonhaltige Styrole, Verfahren zu deren Herstellung und deren Verwendung in elektrophotographischen Aufzeichnungsmaterialien.

Die erfindungsgemäßen sulfonhaltigen Styrole besitzen die allgemeine Formel I

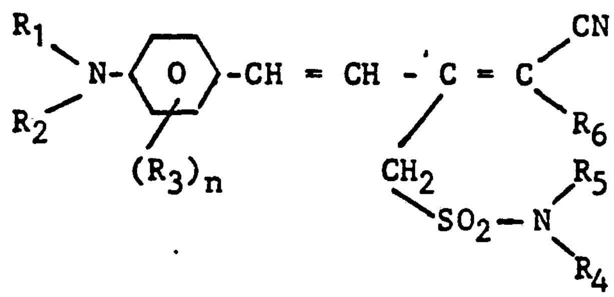

worin $R_1$ und $R_2$ gleich oder verschieden sind und Wasserstoff, gegebenenfalls durch Halogen, Hydroxy-, Cyano- oder ($C_1$ bis $C_4$)-Alkoxy-Gruppen ein- oder mehrfach substituier-Ges ($C_1$ bis $C_8$)-Alkyl oder Phenyl,

$R_3$ Wasserstoff oder eine ($C_1$ bis $C_4$)-Alkyl- -oder -Alkoxy-Gruppe,

n gleich 1 bis 4 oder

$R_1$, $R_2$ und $R_3$ zusammen mit dem zugehörigen Aminophenylrest Julolidinyl oder N-($C_1$ bis $C_3$)-alkylcarbazol-3yl,

$R_4$ und $R_5$ Wasserstoff oder die notwendigen Valenzen zur Bildung eines Kondensationsproduktes mit Formamidderivaten, und

$R_6$ die Cyanogruppe oder

$R_4$ Wasserstoff und $R_5$ und $R_6$ zusammen die Gruppierung

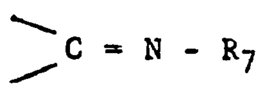 mit

$R_7$ als durch ($C_1$ bis $C_4$)-Alkyl- oder -Alkoxy-Gruppen, Halogen, Cyano-, Amino- oder Mono- oder Di-($C_1$ bis $C_4$)alkylaminogrup-pen substituiertes Benzoyl

bedeuten.

Vorzugsweise werden Verbindungen beansprucht, die in der beigefügten Formeltabelle unter den Nummern II, III, IV und V angegeben sind, bei denen

$R_1$ Methyl, Ethyl oder ω-Chlorethyl,

$R_2$ Methyl oder Ethyl,

$R_3$ Wasserstoff oder Methoxyl,

n gleich 1 oder

$R_1$, $R_2$ und $R_3$ zusammen mit dem zugehörigen Aminophenylrest Julolidinyl,

$R_4$ und $R_5$ Wasserstoff und

$R_6$ die Cyanogruppe bzw.

$R_4$und $R_5$ zusammen die Dimethylaminomethin- oder die 4,6-Dimethylpyrimidin-2-yl-aminomethin-Gruppen darstellen, bzw.

$R_4$ Wasserstoff uni $R_5$ und $R_6$ zusammen die Gruppierung $C=N-R_7$

und

$R_7$ den unsubstituierten oder durch eine Methoxy- oder Cyanogruppe substituierten Benzoylrest

bedeuten.

Die erfindungsgemäßen Verbindungen der Formel II entstehen durch Reaktion der Ketosultame der Formel VI mit Malonsäuredinitril der Formel VII, dargestellt in der beigefügten Reaktionsgleichung 1.

Gegenstand der Erfindung ist auch ein Verfahren zur Herstellung der erfindungsgemäßen sulfonhaltigen Styrole der allgemeinen Formel I nach Anspruch 1, das dadurch gekennzeichnet ist, daß man Verbindungen der allgemeinen Formel VI

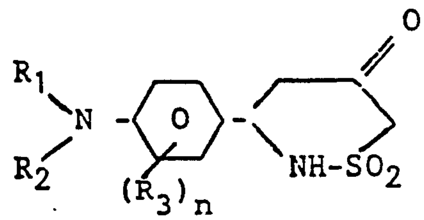

mit den in Anspruch 1 angegebenen Bedeutungen für $R_1$, $R_2$, $R_3$ und n mit Malonsäuredinitril der Formel VII in einem inerten Lösungsmittel bei Temperaturen in einem Bereich von 30 bis 150°C umsetzt, die erhaltenen Verbindungen der allgemeinen Formel II durch Filtration isoliert und gegebenenfalls durch Umkristallisieren reinigt.

Die Umsetzung der Ketosultame der Formel VI mit Malonsäuredinitril der Formel VII wird durch Erhitzen der Komponenten in einem inerten Lösungsmitteldurchgeführt, wobei das dabei gebildete Reaktionswasser durch azeotrope Destillation aus der Reaktionsmischung entfernt werden kann. Geeignete Lösungsmittel sind Alkohole wie Methanol, Ethanol, Propanol und Butanol, chlorierte Kohlenwasserstoffe wie Di-, Tri- und Tetrachlormethan, Ether wie Diethylether, Dioxan und Tetrahydrofuran und Aromaten wie Benzol, Toluol, Xylole und Chlorbenzole. Bevorzugte Lösungsmittel sind Benzol oder Toluol. Die Reaktionstemperatur liegt in einem Bereich von 30 bis 150°C, bevorzugt sind Temperaturen von 70 bis 100°C. Die Verbindungen der Formel VI kristallisieren gewöhnlich aus der Reaktionsmischung aus. Sie können durch Filtration isoliert und durch die üblichen Verfahren, wie zum Beispiel Umkristallisation, gereinigt werden. Sie fallen dann in Form violett- bis tiefblaugefärbter Kristalle an.

Es war nicht vorherzusehen und ist deshalb außerordentlich überraschend, daß durch Umsetzung der ungefärbten Ketosultame der Formel VI mit Malonsäuredinitril der Formel VII nach beigefügter Reaktionsgleichung 1 Farbstoffe der Formel I entstehen, die man zum Beispiel als Sensibilisatoren für elektrophotographische Aufzeichnungsmaterialien verwenden kann.

In Ausgestaltung der Erfindung können die sulfonhaltigen Styrolderivate der Formel II durch Reaktionen an der Sulfonamidgruppe in weitere Farbstoffe überführt werden.

Das abgewandelte Verfahren ist dadurch gekennzeichnet, daß man Verbindungen der allgemeinen Formel II mit Formamidacetalen der Formel VIII (Reaktionsgleichung 2) in einem inerten Lösungsmittel bei Temperaturen im Bereich von 65 bis 120°C umsetzt, das Reaktionsprodukt nach Formel III isoliert und durch Umkristallisieren reinigt.

Das in Formel VIII der Reaktionsgleichung 2 genannte R hat die Bedeutung eines ($C_1$ oder $C_2$)-Alkylrestes.

Die Reaktion nach Gleichung 2 wird durch Erhitzen der Komponenten in einem inerten Lösungsmittel, welches vorzugsweise aus Methanol, Ethanol, Propanol, Butanol oder Mischungen der Alkohole besteht, durchgeführt. Die Reaktionstemperatur liegt vorzugsweise am Siedepunkt des jeweiligen Lösungsmittels.

Die Verbindungen der Formel III nach Formeltabelle kristalllsieren gewöhnlich aus der Reaktionsmischung aus. Sie können durch Abfiltrieren isoliert und durch Umkristallisation gereinigt werden. Sie fallen dann in Form violett gefärbter Kristalle an.

In weiterer Ausgestaltung der Erfindung können die sulfonhaltigen Styrolderivate der Formel II weitere Abwandlungen erfahren.

Ein solches Verfahren ist dadurch gekennzeichnet, daß man Verbindungen der allgemeinen Formel II mit Orthoameisensäureestern der Formel IX und 2-Amino-4,6-di-alkylpyrimidin der Formel X in einem inerten Lösungsmittel bei Temperaturen im Bereich von 60 bis 140°C umsetzt, das Reaktionsprodukt isoliert und gegebenenfalls durch Umkristallisieren reinigt (Reaktionsgleichung 3). Das in der Reaktionsgleichung angeführte R in Formel IX bedeutet einen ($C_1$ oder $C_2$)-Alkylrest. Als Alkylsubstituent des Pyrimidins nach Formel X dient vorzugsweise Methyl.

Die Verbindungen der Formel IX und X sind bekannt.

Die Reaktion wird durch Erhitzen der Reaktionspartner in einem inerten Lösungsmittel durchgeführt. Geeignete Lösungsmittel sind Alkohole wie Methanol, Ethanol, Propanol und Butanol, Aromaten wie Benzol, Toluol, Xylole und Chlorbenzole oder Eisessig. Eine besondere Ausführungsform der Reaktion besteht darin, den Orthoameisensäureester der Formel IX gleichzeitig als Reaktionskomponente und Lösungsmittel zu verwenden. Die Reaktionstemperaturen liegen zwischen 60 und 140°C, bevorzugt ist ein Temperaturbereich von 80 bis 120°C. Die erfindungsgemäßen Verbindungen der Formel IV kristallisieren gewöhnlich aus der Reaktionsmischung aus. Sie können durch Abfiltrieren isoliert und durch Umkristallisation gereinigt werden. Sie fallen dann in Form violett gefärbter Kristalle an.

In weiterer Ausgestaltung der Erfindung ist das Verfahren zur Herstellung der sulfonhaltigen Styrolderivate dadurch gekennzeichnet, daß man Verbindungen der allgemeinen Formel II gemäß Reaktionsgleichung 4 mit gegebenenfalls substituierten Benzoesäurechloriden der Formel XI in einem inerten Lösungsmittel bei Temperaturen im Bereich von 30 bis 100°C umsetzt, vom Ungelösten abfiltriert, das Lösungsmittel durch Destillation entfernt und das erhaltene Reaktionsprodukt durch Umkristallisieren reinigt.

Die Benzoesäurechloride der Formel XI entsprechend $R_7$ in Anspruch 1 sind bekannt. Der in der Reaktionsgleichung 4 angegebene Substituent R hat dabei die Bedeutung von Wasserstoff oder unabhängig

voneinander einer oder mehrerer ($C_1$ bis $C_4$)-Alkyl- oder -Alkoxygruppen, Halogenreste wie Fluor, Chlor oder Brom oder Cyanooder Amino-, Mono- oder Di-($C_1$ bis $C_4$)alkylaminogruppen.

Die Reaktion wird durch Erhitzen der Reaktionspartner in einem inerten Lösungsmittel vorgenommen. Inerte Lösungsmittel sind dabei chlorierte Kohlenwasserstofe wie Di-, Tri- und Tetrachlormethan, Ether wie Diethylether, Diisopropylether, Dioxan und Tetrahydrofuran, und Aromaten wie Benzol, Toluol, Xylole und Chlorbenzole. Die Reaktionstemperatur liegt in einem Bereich zwischen 30 und 100°C. Es ist zweckmäßig, den bei der Reaktion entstehenden Chlorwasserstoff mit einer Base zu neutralisieren. Die Base kann anorganisch sein, wie zum Beispiel die Hydroxide oder Oxide von Alkali- und Erdalkalimetallen, also zum Beispiel Natrium-, Kalium-, Magnesium-, Calcium- und Bariumhydroxid oder Magnesium- oder Calciumoxid, oder organisch sein wie alkylierte Amine, wie zum Beispiel Triethylamin, Diisopropylamin, N-N-Dimethylanilin oder Pyridin und Lutidin.

Die erfindungsgemäßen Verbindungen der Formel V bleiben gewöhnlich in der Reaktionsmischung gelöst. Sie werden isoliert, indem von ungelösten Bestandteilen abfiltriert und das Lösungsmittel abdestilliert wird. Nach Reinigung, wie etwa durch Umkristallisation, fallen sie in Form roter bis violettgefärbter Kristalle an.

Das zur Erzeugung der erfindungsgemäß hergestellten sulfonhaltigen Styrolderivate benötigte Ketosultam der Formel VI ist neu und Gegenstand der am gleichen Tag eingereichten Patentanmeldung, Aktenzeichen P 3 506 435.8 (unser Zeichen Hoe 85/K 017).

Die erfindungsgemäßen sulfonhaltigen Styrolderivate können als Farbstoff oder in elektrophotographischen Aufzeichnungsmaterialien als Sensibilisatoren verwendet werden.

Die Erzeugung von Ladungsbildern auf elektrophotographischen Schichten erfolgt nach der Aufladung durch einen Belichtungsschritt. Durch Belichtung wird zunächst elektrische Ladung erzeugt, die in einem weiteren Schritt durch die Schicht hindurch bis an die Schichtoberfläche transportiert wird und dort vorhandene Ladung neutralisiert Dabei unterscheidet man Photorezeptoren, bei denen Ladungserzeugung und Ladungstransport durch die gleiche chemische Substanz bewirkt werden, von solchen, bei denen die Ladungserzeugung durch Zugabe einer zweiten Substanz, des Sensibilisators, erreicht wird. Dadurch wird die Möglichkeit gegeben, Elektrophotographie auch mit Licht einer Wellenlänge zu betreiben, das von der eigentlich photoleitenden Substanz nicht absorbiert wird. Als ladungstransportierende Verbindungen werden in den zuletzt genannten Photorezeptoren meist organische Donatoren eingesetzt, z.T. auch als Bestandteil eines Polymermoleküls. Zur Sensibilisierung eignen sich sowohl Farbpigmente (zum Beispiel bekannt aus DE-PS-2 108 958, entsprechend US-PS-3 879 200), als auch gelöste Farbstoffe. Als besonders wirksame gelöste Farbstoffe werden Triphenylmethanfarbstoffe, wie zum Beispiel Brillantgrün, C.I. 42 040 (DE-OS-2 817 428, entsprechend US-PS-4 252 880), Rhodaminfarbstoffe, wie zum Beispiel Rhodamin B, C.I. 45 140, und Cyaninfarbstoffe, wie Astrazonorange R oder G, C.I. 48 040, 48 035, (DE-OS-2 949 826, entsprechend AU-PS-539 930), beschrieben.

Die bei in der Polymermatrix gelösten Farbstoffen zu beobachtende geringe Farbechtheit erlaubt nicht den Einsatz als Sensibilisator in cyclisch arbeitenden elektrophotographischen Systemen. Die relativ geringe erforderliche Konzentration und die Wasserlöslichkeit dieser Sensibilisatoren erweist sich dagegen bei Anwendung in elektrophotographischen Druckplatten und gedruckten Schaltungen als Vorteil.

Nachteilig auch bei dieser Art der Anwendung ist jedoch die in diesen Systemen beobachtete hohe Vorbelichtungsempfindlichkeit. Wird ein mit löslichen Farbstoffen der genannten Art sensibilisierter elektrophotographischer Photorezeptor vor dem Kopierprozeß versehentlich Licht ausgesetzt, so wird nach dem Aufladen mittels einer Corona auf der zuvor belichteten Stelle eine stark verminderte Ladungsannahme beobachtet. Erst durch Lagern während mehrerer Stunden wird dieser Memory Effekt beseitigt.

Die geringe Löslichkeit der eingesetzten "löslichen" Farbstoffe - es handelt sich durchweg um kationische Farbstoffe - in den zur Beschichtung verwendeten organischen Lösungsmitteln wie Tetrahydrofuran oder Estern erfordert zudem einen zusätzlichen Verarbeitungsschritt, in dem der Farbstoff zunächst in einem Alkohol vorgelöst wird und anschließend als Lösung der fertigen Beschichtungslösung zugesetzt wird.

Schließlich handelt es sich bei einem Teil der bislang verwendeten löslichen Sensibilisierungsfarbstoffe, zum Beispiel Brillantgrün, um Substanzen, deren Verwendung wegen ihrer Wasserlöslichkeit physiologisch nicht unbedenklich ist. Dies erfordert besondere Vorsichtsmaßnahmen beim Umgang mit den diese Farbstoffe enthaltenden Entschichterlösungen.

Es bestand daher ein Bedarf an in der Polymermatrix gelöst vorliegenden Sensibilisierungsfarbstoffen für die Elektrophotographie, die die genannten Nachteile nicht aufweisen.

Dies wird erfindungsgemäß erreicht mit sulfonierten Styrolderivaten der allgemeinen Formel I, in der $R_1$ bis $R_6$ und n die oben angegebene Bedeutung haben.

Diese Sensibilisierungsfarbstoffe werden Photoleiterschichten von elektrophotographischen Aufzeichnungsmaterialien zugesetzt, die aus einem leitenden Schichtträger, gegebenenfalls einer isolierenden Zwischenschicht geringer Schichtdicke, und einer photoleltfähigen Schicht bestehen. Die photoleitfähige Schicht kann als Monoschicht Sensibilisator und Photoleiter in einer Schicht enthalten; sie kann jedoch auch aus einer Ladungsträger erzeugenden und einer Ladungstransport-Schicht bestehen. In diesem Fall kann die Ladungsträger erzeugende Schicht den oder die Sensibilisatoren, ein elektronisch inertes Bindemittel und gegebenenfalls einen oder mehrere organische Photoleiter enthalten, während die Ladungstransportschicht sensibilisatorfrei und als wesentliche Schichtbestandteile Photoleiter und Bindemittel enthalten kann. Der organische Photoleiter kann dabei auch, wie in der deutschen Patentanmeldung, Aktenzeichen P 33 29 442.9) beschrieben, durch einen Diffusionsprozeß in die Ladungsträger erzeugende Schicht eingebracht werden, indem beim Antrag der Ladungstransportschicht ein Lösungsmittel eingesetzt wird, in dem das Bindemittel der

Ladungsträger erzeugenden Schicht löslich oder quellbar ist.

Als elektrisch leitende Schichtträger sind Materialien mit genügend elektrisch leitenden Eigenschaften geeignet, wie sie auch bisher bereits zu diesem Zweck verwendet wurden. Der Schichtträger kann in Form eines flexiblen Bandes oder einer Platte vorliegen. In bevorzugter Ausführungsform ist der Schichtträger für die Herstellung von Druckformen und gedruckten Schaltungen geeignet und besteht zum Beispiel aus einer Aluminium-, Zink-, Magnesium-, Kupfer-, Eisen-, Nickel- oder einer Mehrmetallplatte. Es kommen auch metallis-ierte, zum Beispiel metallbedampfte, Kunststoffolien, wie aluminiumbedampfte Polyesterfolien, oder auch kupferkaschierte Polyimidfolien und -platten in Frage.

Besonders bewährt haben sich oberflächenveredelte Schichtträger aus Aluminium. Die Oberflächenveredlung besteht in einer mechanischen oder elektrochemischen Aufrauhung und gegebenenfalls in einer anschließenden Anodisierung und Behandlung mit Polyvinylphosphonsäure gemäß DE-OS-1 621 478, entsprechend US-PS-4 153 461.

Ganz allgemein kann als isolierende Zwischenschicht eine Sperrschicht wie eine thermisch, anodisch bzw. chemisch erzeugte Metalloxidschicht, zum Beispiel aus Aluminiumoxid, dienen. Die Sperrschicht hat die Aufgabe, die Ladungsträgerinjektion vom elektrisch leitenden Schichtträger im Dunkeln in die Ladungsträger erzeugende Schicht herabzusetzen bzw. zu verhindern. Weiterhin wird durch die Sperrschicht die Haftung der folgenden Schichten auf den Schichtträger günstig beeinflußt. Für organische Sperrschichten können verschiedene Naturoder Kunstharzbindemittel verwendet werden, die gut auf einer Metall- bzw. Aluminiumoberfläche haften und beim nachfolgenden Anbringen der weiteren Schichten keine An- oder Ablösung erfahren. Die Dicke der organischen Sperrschicht liegt im Bereich von 1 µm, die einer Metalloxidschicht in der Größenordnung von 10 bis $10^4$ nm.

Zur Herstellung von beispielsweise gedruckten Schaltungen, wie sie in der Elektronik üblich sind, kann die photoleitfähige Doppelschicht auch zunächst auf einen Zwischenträger aufgebracht werden, von dem aus sie als sogenannter Trockenresist auf den Schichtträger anschließend oder später übertragen wird. Dies kann zum Beispiel durch Laminieren erfolgen. Als Zwischenträger haben sich Kunststoffolien, wie solche aus Polyester, insbesondere aus Polyethylenterephthalatfolie, besonders bewährt.

Als sensibilisierende Farbstoffe finden erfindungsgemäß sulfonierte Styrolderivate der allgemeinen Formel I Verwendung, die gegebenenfalls im Gemisch, auch mit Sensibilisierungsfarbstoffen anderer Farbstoffklassen, eingesetzt werden können, um zum Beispiel den Spektralbereich der Sensibilisierung zu erweitern. Um gute Sensibilisierungseigenschaften zu erzielen, hat es sich bewährt, die Farbstoffe in Konzentrationen zwischen 0,1 und 5 Gew.-%, bevorzugt zwischen 0,25 und 2 Gew.-%, bezogen auf das Gewicht der Photoleiterschicht, zuzusetzen.

Als dem Ladungstransport dienende Verbindungen in der Ladungstransportschicht sind vor allem solche geeignet, die ein ausgedehntes π-Elektronensystem besitzen. Hierzu gehören monomere heterocyclische Verbindungen, die durch dialkylsubstituierte Aminogruppen oder Alkoxygruppen substituiert sind. Bewährt haben sich besonders heterocyclische Verbindungen, wie Oxdiazolderivate, die in der deutschen Patentschrift 10 58 838, entsprechend US-PS-3 189 447, genannt sind. Hierher gehören auch Triphenylamin-Derivate, Oxazol-, Pyrazolin-, Triazolund Imidazol-Derivate, wie sie zum Beispiel aus DE-PS-1 120 875, 1 060 260, 1 060 714 (entsprechend US-PS-3 257 203, 3, 112 197 und 3 180 729) hervorgehen. Auch Hydrazonverbindungen, wie sie zum Beispiel in DE-OS-2 919 791, entsprechend US-PS-4 278 747, genannt sind, können eingesetzt werden. Vorzugsweise werden 2,5-Bis-(4'-diethylaminophenyl)-1,3,4-oxdiazol, p-Methoxybenzaldehyddiphenylhydrazon und/oder [ ,5-Diphenyl-3-p-methoxyphenylpyrazolin verwendet.

Das hochisolierende Bindemittel für die ladungsträgererzeugende Schicht und für die Ladungstransportschicht kann gleich oder unterschiedlich sein. Als solche sind hinsichtlich der Flexibilität, der Filmeigenschaften und der Haftfestigkeit Natur- und Kunstharze geeignet, die sich durch gebräuchliche Lösungsmittel oder Lösungsmittelgemische bei der Herstellung der Schichten anlösen bzw. anquellen lassen. Hierzu gehören Polyesterharze, die Mischpolyester aus Iso- und Terephthalsäure mit Glykolen darstellen. Auch Silikonharze haben sich als geeignet erwiesen. Polycarbonatharze sind gut einsetzbar. Besonders bevorzugt für die Herstellung von Druckformen und gedruckten Schaltungen sind Bindemittel, die in wäßrigen oder alkoholischen Lösungsmittelsystemen, gegebenenfalls unter Säure- oder Alkalizusatz, löslich sind. Aus physiologischen und Sicherheitsgründen scheiden aromatische oder aliphatische, leicht brennbare Lösungsmittel aus. Geeignete Harzbindemittel sind hiernach hochmolekulare Substanzen, die alkalilöslich machende Gruppen tragen. Solche sind beispielsweise Säureanhydrid-, Carboxyl-, Carbonsäureamid-, Phenol-, Sulfonsäure-, Sulfonamid- oder Sulfonimid-Gruppen. Bevorzugt werden Harzbindemittel mit hohen Säurezahlen eingesetzt. Mischpolymerisate mit Anhydridgruppen können mit gutem Erfolg verwendet werden, da durch das Fehlen freier Säuregruppen die Dunkelleitfähigkeit gering ist, trotz guter Alkalilöslichkeit. Besonders bewährt haben sich Copolymerisate aus Styrol und Maleinsäureanhydrid, Sulfonylurethane gemäß DE-OS-3 210 577, und Copolymerisate der Acryl- bzw. Methacrylsäure.

Als übliche Zusätze enthalten die Schichten Substanzen, die der Beschichtungslösung zugesetzt werden und dadurch die Oberflächenstruktur und die Flexibilität verbessern. Dies können zum Beispiel Weichmacher, wie Triphenylphosphat, oder Verlaufmittel, wie Silikonöle, sein.

Die Beschichtungen bringt man in üblicher Weise auf, zum Beispiel durch Rakel- oder Sprühantrag. Vorzugsweise wird der Antrag mit einem Fließer vorgenommen. Die Trocknung der Schichten erfolgt beispielsweise in Trokkenkanälen, wobei die verschiedenen Trocknungsstufen durch die Temperatur der einzelnen Bereiche, durch die Laufgeschwindigkeit des Materials und durch den herrschenden Luftdurchsatz festgelegt werden.

**Experimenteller teil**

Die Verbindungen der in Tabelle 1 aufgeführten Beispiele 1 bis 4, welche die Formel II der Formeltabelle besitzen, wurden nach folgender allgemein anwendbarer Methode hergestellt:

Jeweils 50 mmol einer Verbindung der Formel VI, hergestellt nach der am gleichen Tag eingereichten deutschen Patentanmeldung, Aktenzeichen P (unser Zeichen Hoe 85/K 017), und 50 mmol Malonsäuredinitril wurden in 100 ml Benzol unter azeotroper Entfernung des Reaktionswassers am Wasserabscheider erhitzt. Nach beendeter Reaktion ließ man abkühlen, isolierte das Produkt durch Abfiltrieren und kristallisierte aus Acetonitril bzw. Eisessig um.

**Tabelle 1**

| Beispiel Nr. | $R_1$ | $R_2$ | $R_3$ | n | Fp (°C) | Ausbeut4 | $\lambda_{max}$(DMF) (ε) |
|---|---|---|---|---|---|---|---|
| 1 | ω-Chlorethyl | Methyl | H | | 208-216 | 56 % | 502 nm (15300) |
| 2 | Methyl | Methyl | 2-Methoxyl | 1 | 215-220 | 43 % | 522 nm (19300) |
| 3 | Ethyl | Ethyl | H | | 212-216 | 38 % | 525 nm (23400) |
| 4 | Julolidinyl | | | | 227-231 | 47 % | 557 nm (21 200) |

Die Verbindungen der in Tabelle 2 aufgeführten Beispiele 5 bis 7, welche die Formel III der Formeltabelle besitzen, wurden nach folgender allgemein anwendbarer Methode hergestellt:

50 mmol einer Verbindung der in Tabelle 1 genannten Beispiele und 60 mmol Dimethylformamiddimethylacetal wurden in 100 ml isopropanol während fünf Stunden unter Rückfluß gerührt. Man kühlte und filtrierte das Produkt ab, das durch Kristallisation aus Eisessig gereinigt wurde.

**Tabelle 2**

| Beispiel Nr. | $R_1$ | $R_2$ | $R_3$ | Fp (°C) | Ausbeute | $\lambda_{max}$(DMF) (ε) |
|---|---|---|---|---|---|---|
| 5 | ω-Chlorethyl | Methyl | H | 216-218 | 51 % | 505 nm (27000) |
| 6 | Ethyl | Ethyl | H | 198-202 | 50 % | 525 nm (46300) |
| 7 | Julolidinyl | | | 241-243 | 33 % | 559 nm (47600) |

**Beispiel 8**

17 g (50 mmol) der Verbindung des Beispiels 3, 12,3 g (50 mmol) 2-Amino-4,6-dimethyl-pyrimidin und 50 ml Orthoameisensäureethylester wurden fünf Stunden auf 100°C erhitzt. Man ließ abkühlen und isolierte durch Abfiltrieren 20 g Rohprodukt. Es ergaben sich nach Umkristallisation 9,1 g einer violett gefärbten Verbindung entsprechend der Formel IV der Formeltabelle mit den Daten:

Fp 168-171°C, Ausbeute 42 %,
$\lambda_{max}$(DMF) = 530 nm, ε = 23900.

Die Beispiele 9 bis 13 der Tabelle 3, welche die Formel V der Formeltabelle besitzen, wurden nach folgender allgemein anwendbarer Methode hergestellt;

50 mmol einer Verbindung der in Tabelle 1 genannten Beispiele und 60 mmol eines entsprechendem Benzoesäurechlorids wurden in 100 ml Tetrahydrofuran auf 50°C erwärmt. Dazu tropfte man 80 mmol Triethylamin. Nach sechs Stunden ließ man abkühlen, filtrierte vom Ungelösten und dampfte das Lösungsmittel ab. Der Rückstand lielß sich aus Eisessig umkristallisieren

**Tabelle 3**

| Beispiel Nr. | $R_1$ | $R_2$ | $R_3$ | R | Fp (°C) | Ausbeute | $\lambda_{max}$(DMF) (ε) |
|---|---|---|---|---|---|---|---|
| 9 | ω-Chlorethyl | Methyl | H | H | 202-204 | 54 % | 482 nm (18700) |
| 10 | Ethyl | Ethyl | H | H | 224 | 42 % | 507 nm (49200) |
| 11 | Ethyl | Ethyl | H | Meth-oxyl | 222-224 | 40 % | 501 nm (26300) |
| 12 | Ethyl | Ethyl | H | Cyano | 240-243 | 72 % | 509 nm (43300) |
| 13 | Julolidinyl | | | H | 220-222 | 34 % | 537 nm (45500) |

Für die in den Beispielen 1 bis 13 genannten Verbindungen ergab die Elementaranalyse folgende Werte:

**Tabelle 4**

| Beispiel Nr. | Summenformel (Molmasse) | Ber. Gef. | C | H | N | S |
|---|---|---|---|---|---|---|
| 1 | $C_{16}H_{15}N_4O_2SCl$ (364,84) | | 52,7<br>52,4 | 4,3<br>4,2 | 15,4<br>15,6 | 8,8<br>8,7 |
| | | Cl: | Ber. 9,6 | | Gef. 9,5 | |
| 2 | $C_{16}H_{18}N_4O_3S$ (346,52) | | 55,5<br>55,4 | 5,2<br>5,3 | 16,2<br>16,2 | 9,2<br>9,1 |
| 3 | $C_{16}H_{20}N_4O_2S$ (344,43) | | 59,4<br>59,0 | 5,9<br>5,9 | 16,3<br>16,0 | 9,3<br>9,6 |
| 4 | $C_{18}H_{20}N_4O_2S$ (368,42) | | 62,8<br>62,7 | 5,4<br>5,3 | 15,2<br>15,2 | 8,7<br>8,8 |
| 5 | $C_{19}H_{22}N_5O_2SCl$ (419,30) | | 54,4<br>54,1 | 5,3<br>5,2 | 16,7<br>16,9 | 7,6<br>7,5 |
| | | Cl: | Ber. 8,4 | | Gef. 8,6 | |
| 6 | $C20H_{25}N_5O_2S$ (399,51) | | 60,2<br>60,1 | 6,3<br>6,2 | 17,5<br>17,2 | 8,0<br>7,9 |
| 7 | $C_{22}H_{25}N_5O_2S$ (433,53) | | 62,4<br>62,4 | 5,9<br>5,8 | 16,5<br>16,2 | 7,6<br>7,8 |
| 8 | $C_{24}H_{28}N_7O_2S$ (477,57) | | 60,3<br>60,2 | 5,9<br>5,7 | 20,5<br>20,5 | 6,7<br>6,5 |
| 9 | $C_{23}H_{21}N_4O_3SCl$ (468,9) | | 58,9<br>59,0 | 4,5<br>4,5 | 11,9<br>11,7 | 6,8<br>6,6 |
| | | Cl: | Ber. 7,5 | | Gef. 7,3 | |
| 10 | $C_{24}H_{24}N_4O_3S$ (448,5) | | 64,3<br>64,3 | 5,4<br>5,4 | 12,5<br>12,3 | 7,2<br>7,7 |

| | | | | | |
|---|---|---|---|---|---|
| 11 | $C_{25}H_{26}N_4O_4S$ (478,57) | 62,7<br>62,8 | 5,5<br>5,5 | 11,7<br>11,5 | 6,7<br>6,4 |
| 12 | $C_{25}H_{23}N_5O_3S$ (473,55) | 63,4<br>63,2 | 4,9<br>4,8 | 14,8<br>14,6 | 6,8<br>6,5 |
| 13 | $C_{26}H_{24}N_4O_3S$ (472,57) | 66,1<br>66,3 | 5,1<br>5,2 | 11,9<br>12,0 | 6,8<br>6,6 |

**Anwendungsbeispiele**

**Beispiele I bis XII**

Je

0,5 g der Verbindungen der Tabellen 1 bis 3
, 50 g 2,5 Bis(4-diethylaminophenyl)1,3,4-oxdiazol
und
50 g eines Sulfonylurethans, hergestellt durch Umsetzung eines Polyvinylbutyrals mit - bezogen auf freie OH-Gruppen äquimolarer Menge - Propenylsulfonylisocyanat, beschrieben in der DE-OS-3 210 577, Beispiel 1,

wurden in

900 g Tetrahydrofuran

gelöst. Nach Zugabe von 0,1 g eines Silikonöls der Viskosität 5 bis 20 mPa.s wurde diese Beschichtungslösung auf einen elektrochemisch vorbehandelten und anodisierten Aluminiumträger, wie er zur Herstellung von Offsetdruckplatten üblicherweise verwendet wird, so aufgetragen, daß ein Trockenschichtgewicht von 6 g/m² resultierte.

Die so hergestellten vorsensibilisierten elektrophotographischen Druckplatten zeigten die in Tabelle 5 wiedergegebenen elektrophotographischen Eigenschaften. Sie ließen sich mit wäßrig-alkalischen Entschichtern grundfrei entschichten. Bei einem Druckversuch ergab eine auf diese Art hergestellte Druckplatte eine Auflage von weit über 100.000 bei guter Tonwertwiedergabe.

**Beispiel XIII**

0,5 g der Verbindung aus Beispiel 3 und
49,5 g des Sulfonylurethans wurden in
450 g Tetrahydrofuran

gelöst und auf einen elektrochemisch vorbehandelten und anodisierten Aluminiumträger so aufgetragen, daß ein Schichtgewicht von 3 $g/m^2$ resultierte. Auf diese Ladungsträger erzeugende Schicht wurde eine Ladungstransportschicht aus

25,0 g 2,5-Bis-(4-diethylaminophenyl)-1,3,4-oxdiazol,
25,0 g Sulfonylurethan,
0,1 g Silikonöl und
450 g Tetrahydrofuran

so aufgetragen, daß das Gesamtschichtgewicht der Doppelschicht 6 $g/m^2$betrug.

Die elektrophotographischen Daten sind, wie auch die der folgenden Beispiele, der Tabelle 5 zu entnehmen.

**Beispiel XIV**

Es wurde vorgegangen wie in Beispiel V, mit dem Unterschied, daß anstelle des Sulfonylurethans ein alternierendes Copolymerisat aus Styrol und Maleinsäureanhydrid (Zers.punkt 200 bis 240° C) verwendet wurde.

Anstelle des 2,5-Bis-(p-diethylaminophenyl)-1,3,4-oxdiazols wurde 1,5-Diphenyl-3-p-methoxy-phenyl-pyrazolin, analog DE-AS 1 060 714, verwendet.

**Beispiel XV**

Es wurde vorgegangen wie in Beispiel V, mit dem Unterschied, daß anstelle des 2,5-Bis-(p-diethylaminophenyl)-1,3,4-oxdiazols p-Methoxybenzaldehyd-diphenylhydrazon gemäß DE-OS-2 919 791 und anstelle des Sulfonylurethans ein Terpolymerisat aus Styrol, n-Hexylmethacrylat und Methacrylsäure im Molverhältnis 10 : 60 : 30 verwendet wurde.

**Beispiel XVI**

Es wurde verfahren wie in Beispiel II, mit dem Unterschied, daß die photoleitfähige Schicht zunächst auf eine Polyethylenterephthalatfolie als Zwischenträger aufgetragen und in einem Laminierschritt von diesem auf einen Cu-kaschierten Polyimidträger übertragen wurde.

**Beispiel XVII** (Vergleichsbeispiel)

Es wurde vorgegangen wie in Beispiel II, mit dem Unterschied, daß anstelle des Farbstoffes aus Beispiel 3 Astrazonorange R (C.I. 48040) verwendet wurde. Dieser Farbstoff wurde vor der Zugabe zu der Beschichtungslösung in Methanol gelöst.

**Beispiel XVIII** (Vergleichsbeispiel)

Es wurde vorgegangen wie in Beispiel II, mit dem Unterschied, daß anstelle des Farbstoffes aus Beispiel 3 der Laserfarbstoff Nr. 17 (DE-AS-2 831 054, entsprechend US-PS-4 145 215) mit der Formel

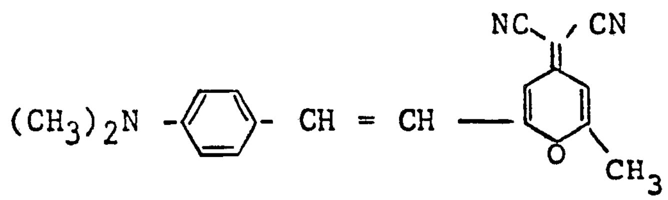

verwendet wurde.

**Beispiel XIX** (Vergleichsbeispiel)

Es wurde vorgegangen wie in Beispiel XVII, mit dem Unterschied, daß außer dem Astrazonorange zusätzlich die gleiche Menge Rhodamin FB (C.I. 45 170) der Schicht zugesetzt wurde.

Die Ergebnisse der Anwendungsbeispiele I bis XIX sind in der folgenden Tabelle 5 zuzsammengestellt. Hieraus entnimmt man, daß Aufzeichnungsmaterialien mit den erfindungsgemäßen sulfonhaltigen Styrolderivaten bei guten elektrophotographischen Eigenschaften eine ganz ausgezeichnete Vorbelichtungsunempfindlichkeit besitzen, was für die technische Handhabung von herausragender Bedeutung ist.

**Tabelle 5**

| Anwendungs-beispiel Nr. | | Farbstoff aus Bei-spiel Nr. | Ladungs-annahme $U_{max}$ | Dunkel-abfall $U_{dkl}$ (60") | $E_{1/2}$+ | $U_o$ nach Vorbeliche-tung++ |
|---|---|---|---|---|---|---|
| I | | 1 | -750 | -722 | 13,4 | -735 |
| II | | 3 | -800 | -793 | 11,7 | -742 |
| III | | 4 | -869 | -790 | 22,6 | -788 |
| IV | | 5 | -687 | -560 | 13,6 | -643 |
| V | | 6 | -951 | -861 | 10,7 | -790 |
| VI | | 7 | -671 | -541 | 19,9 | -643 |
| VII | | 8 | -707 | -668 | 13,4 | -683 |
| VIII | | 9 | -616 | -462 | 11,4 | -513 |
| IX | | 10 | -944 | -876 | 10,9 | -841 |
| X | | 11 | -699 | -572 | 15,9 | -628 |
| XI | | 12 | -596 | -355 | 15,8 | -484 |
| XII | | 13 | -651 | -489 | 15,8 | -584 |
| XIII | | 3 | -750 | -735 | 12,3 | -728 |
| XIV | | 6 | -869 | -807 | 25,6 | -807 |
| XV | | 6 | -1007 | -935 | 17,1 | -734 |
| XVI | | 3 | -755 | -754 | 10,9 | -734 |
| II | (Vergl.) | - | -711 | -668 | 16,3 | -537 |
| XVIII | " | - | -742 | -699 | 24,6 | - |
| XIX | " | - | -766 | -722 | 8,2 | -418 |

\+ erforderliche Lichtenergie zur Entladung des Photorezeptors auf die Hälfte der ursprünglichen Aufladung, in uJ/cm² bei einer Aufladung auf -400 V und Belichtung mit einer Halogenwolframlampe unter Verwendung von Kantenfiltern bei 700 nm.

++ maximale Ladungsannahme nach Belichten mit Leuchtstoffröhren (1000 lux) während 10 min.

**Formeltabelle**

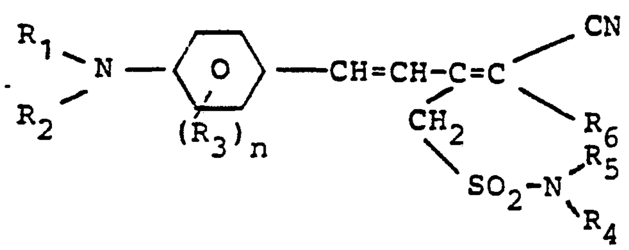

I

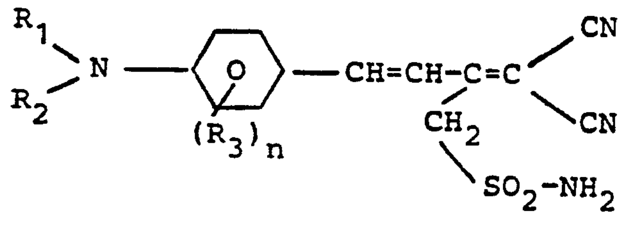

II

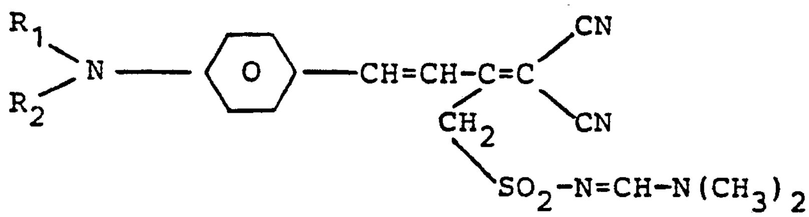

III

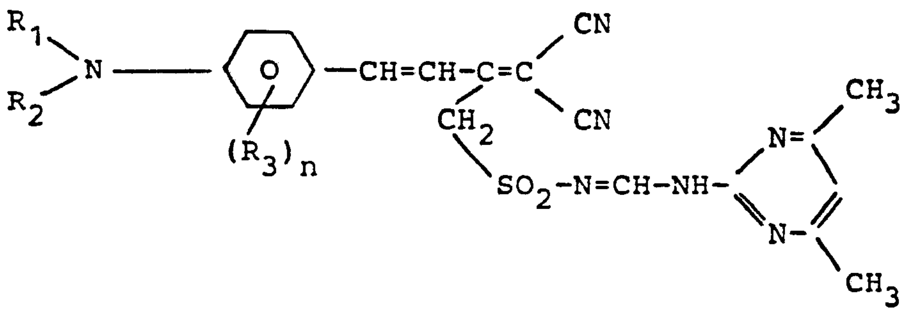

IV

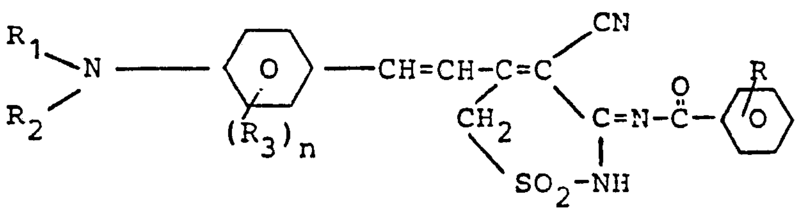

V

**Reaktionsgleichungen**

1. 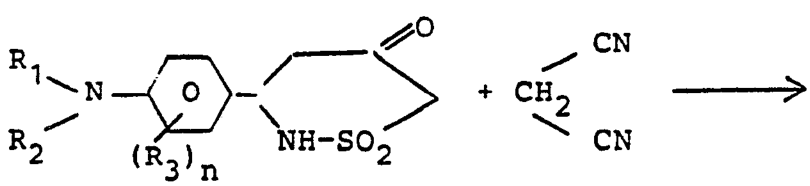 II

VI VII

2. II + $(RO)_2CH-N(CH_3)_2$ → III

VIII

3. 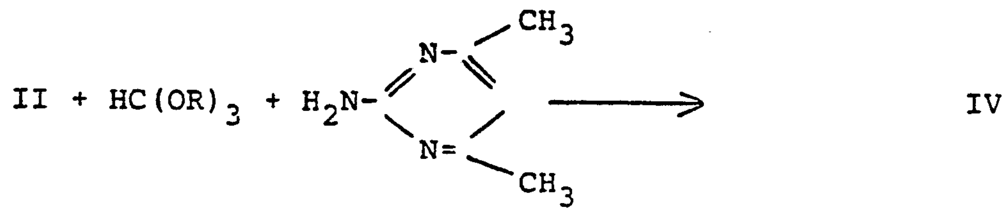

IX X

4. 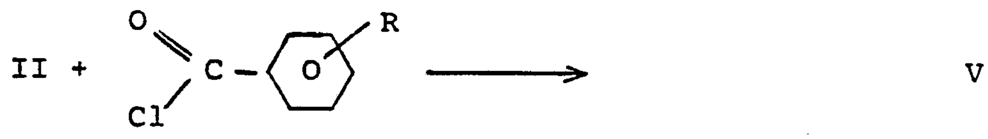

XI

## Patentansprüche

1. Neue sulfonhaltige Styrolderivate der allgemeinen Formel I

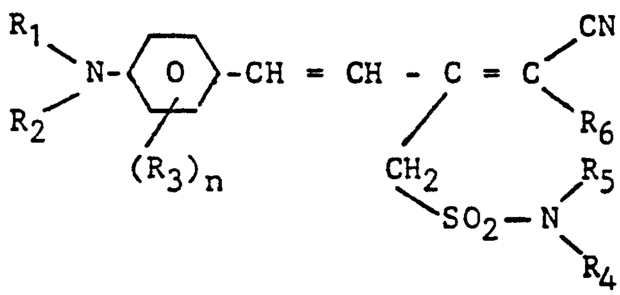

worin $R_1$ und $R_2$ gleich oder verschieden sind und Wasserstoff, gegebenenfalls durch Halogen, Hydroxy-, Cyano- oder( $C_1$ bis $C_4$)-Alkoxy-Gruppen ein- oder mehrfach substituiertes ($C_1$ bis $C_4$)Alkyl oder Phenyl,
$R_3$ Wasserstoff oder eine ($C_1$ bis $C_4$)-Alkyl-oder -Alkoxy-Gruppe,
n gleich 1 bis 4 oder
$R_1$, $R_2$ und $R_3$ zusammen mit dem zugehörigen Aminophenylrest Julolidinyl oder N-($C_1$ bis $C_3$)-alkylcarbazol-3-yl,
$R_4$ und $R_5$ Wasserstoff oder die notwendigen Valenzen zur Bildung eines Kondensationsproduktes mit Formamidderivaten, und
$R_6$ die Cyanogruppe oder
$R_4$ Wasserstoff und $R_5$ und $R_6$ zusammen die Gruppierung

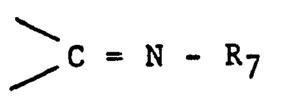 mit

$R_7$ als durch ($C_1$ bis $C_4$)-Alkyl- oder -Alkoxy-Gruppen, Halogen, Cyano-, Amino- oder Mono- oder Di-($C_1$ bis $C_4$)alkylaminogrup-pen substituiertes Benzoyl
bedeuten.

2. Styrolderivat nach Anspruch 1, dadurch gekennzeichnet, daß $R_1$ Methyl, Ethyl oder ω-Chlorethyl,
$R_2$ Methyl oder Ethyl,
$R_3$ Wasserstoff oder Methoxyl,
n gleich 1 oder
$R_1$, $R_2$, $R_3$ zusammen mit dem zugehörigen Aminophenylrest Julolidinyl,
$R_4$ und $R_5$ Wasserstoff und
$R_6$ die Cyanogruppe
bedeuten.

3. Styrolderivate nach Anspruch 1, dadurch gekennzeichnet, daß $R_4$ und $R_5$ zusammen die Dimethylaminomethingruppe darstellen.

4. Styrolderivat nach Anspruch 1, dadurch gekennzeichnet, daß $R_4$ und $R_5$ zusammen die 4,6-Dimethylpyrimidin-2-yl-aminomethingruppe darstellen.

5. Styrolderivat nach Anspruch 1, dadurch gekennzeichent, daß $R_4$ Wasserstoff und $R_5$ und $R_6$ zusammen die Gruppierung

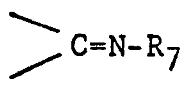

und $R_7$ den unsubstituierten oder durch eine Methoxy- oder Cyanogruppe substituierten Benzoylrest darstellen.

6. Verfahren zur Herstellung der sulfonhaltigen Styrole der allgemeinen Formel I nach Anspruch 1, dadurch gekennzeichnet, daß man Verbindungen der allgemeinen Formel VI

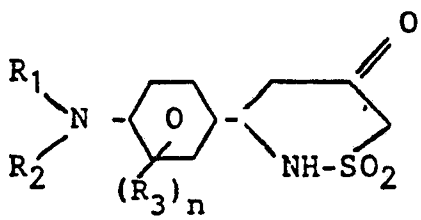

mit den in Anspruch 1 für $R_1$, $R_2$, $R_3$ und n angegebenen Bedeutungen mit Malonsäuredinitril in einem inerten Lösungsmittel bei Temperaturen im Bereich von 30 bis 150°C umsetzt, die erhaltenen Verbindungen der allgemeinen Formel II durch Filtration isoliert und gegebenenfalls durch Umkristallisieren reinigt.

7. Verfahren nach Anspruch 6, dadurch gekennzeichnet, daß man Verbindungen der allgemeinen Formel II mit Formamidacetalen der Formel VIII (Reaktionsgleichung 2) in einem Lösungsmittel bei Temperaturen im Bereich von 65 bis 120°C umsetzt, das Reaktionsprodukt nach Formel III isoliert und durch Umkristallisieren reinigt.

8. Verfahren nach Anspruch 6, dadurch gekennzeichnet, daß man Verbindungen der allgemeinen Formel II mit Orthoameisensäureestern der Formel IX und 2-Amino-4,6-dialkylpyrimidin der Formel X (Reaktionsgleichung 3) in einem inerten Lösungsmittel bei Temperaturen im Bereich von 60 bis 140°C umsetzt, das Reaktionsprodukt isoliert und gegebenenfalls durch Umkristallisieren reinigt.

9. Verfahren nach Anspruch 6, dadurch gekennzeichnet, daß man Verbindungen der allgemeinen Formel II mit gegebenenfalls substituierten Benzoesäurechloriden der Formel XI (Reaktionsgleichung 4) in einem inerten Lösungsmittel bei Temperaturen im Bereich von 30 bis 100°C umsetzt, vom Ungelösten abfiltriert, das Lösungsmittel durch Destillation entfernt und das erhaltene Reaktionsprodukt durch Umkristallisieren reinigt.

10. Verwendung der sulfonhaltigen Styrolderivate nach Ansprüchen 1 bis 5 als Farbstoffe.

11. Verwendung der sulfonhaltigen Styrolderivate nach Ansprüchen 1 bis 5 als Sensibilisatoren in elektrophotographischen Aufzeichnungsmaterialien.

12. Verwendung der Styrolderivate nach Ansprüchen 1 und 11 zur Herstellung von Druckformen oder gedruckten Schaltungen.

## Claims

1. New sulfonyl-containing styrene derivatives of the general formula I

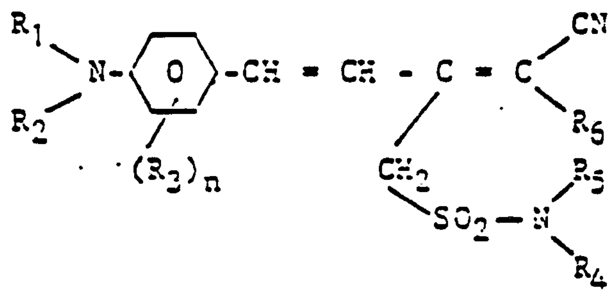

in which $R_1$ and $R_2$ are identical or different and denote hydrogen, optionally halogen-, hydroxyl-, cyano- or $(C_1\text{-}C_4)$-alkoxy-mono- or di-substituted $(C_1\text{-}C_8)$-alkyl or phenyl,

$R_3$ denotes hydrogen or a ($C_1$-$C_4$)-alkyl or -alkoxy group,
n is equal to 1 to 4 or
$R_1$, $R_2$ and $R_3$ together with the associated aminophenyl radical denote julolidinyl or N-($C_1$-$C_3$)-alkylcarbazol-3-yl,
$R_4$ and $R_5$ denote hydrogen or the necessary valences for forming a condensation product with formamide derivatives, and
$R_6$ denotes a cyano group or
$R_4$ denotes hydrogen and $R_5$ and $R_6$ together denote the grouping

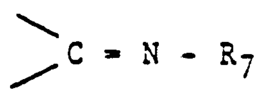

with
$R_7$ as benzoyl which is substituted by ($C_1$-$C_4$)-alkyl or -alkoxy groups, halogen, cyano, amino or mono- or di-($C_7$-$C_4$)-alkylamino groups.

2. Styrene derivative, as claimed in claim 1, wherein
$R^1$ denotes methyl, ethyl or ω-chloroethyl,
$R_2$denotes methyl or ethyl,
$R_3$ denotes hydrogen or methoxyl,
n is - equal to 1 or
$R_1$, $R_2$, $R_3$ together with the associated aminophenyl radical denote julol idinyl,
$R_4$ and $R_5$ denote hydrogen and
$R_6$ denotes a cyano group.

3. Styrene derivative as claimed in claim 1, wherein $R_4$ and $R_5$ together represent a dimethylaminomethine group.

4. Styrene derivative as claimed in claim 1, wherein $R_4$ and $R_5$ together represent a 4,6-dimethylpyrimidin 2-ylaminomethine group.

5. Styrene derivative as claimed in claim 1, wherein $R_4$ represents hydrogen and $R_5$ and $R_6$ together represent the grouping

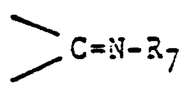

and $R_1$ represents a benzoyl radical which is unsubstituted or substituted by a methoxy or cyano group.

6. A process for preparing the sulfonylcontaining styrenes of the formula I as claimed in claim 1, which comprises reacting compounds of the general formula VI

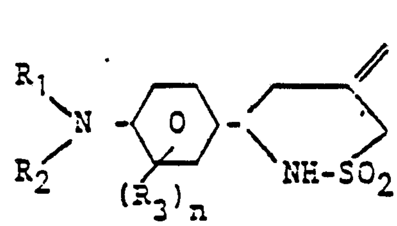

where $R_1$, $R_2$, $R_3$ and n have the meanings specified in claim 1 with malonitrile in an inert solvent at temperatures within the range from 30 to 150°C, isolating the resulting compounds of the formula II by filtration and where appropriate purifying by recrystallization.

7. The process as claimed in claim 6, wherein compounds of the genenral formula II are reacted with formamide acetals of the formula VIII (reaction equation 2) in a solvent at temperatures within the range from 65 to 120°C, the reaction product of the formula III is isolated and then purified by recrystallization.

8. The process as claimed in claim 6, wherein a compound of the formula II are reacted with orthoformic acid esters of the formula IX and 2-amino-4,6-dialkylpyrimidine of the formula X (reaction equation 3) in an inert solvent at temperatures within the range from 60 to 140°C, the reaction product is isolated and where appropriate purified by recrystallization.

9. The process as claimed in claim 6, where in a compound of the formula II is reacted with optionally substituted benzoic acid chloride of the formula XI (reaction equation 4) in an inert solvent at temperatures with in the range from 30 to 100°C, undissolved matter is filtered off, the solvent is removed by distillation, and the resulting reaction product is purified by recrystallization.

10. Use of the sulfonylcontaining styrene derivative as claimed in claims 1 to 5, as dyes.

11. Use of the sulfonyl-containing styrene derivative as claimed in claims 1 to 5, as sensitiers in electrophotographic recording materials.

12. Use of the styrene derivatives as claimed in claims and 11, for preparing printing forms or printed circuits.

**Revendications**

1. Nouveaux dérivés de styrene sulfonés de formule générale I

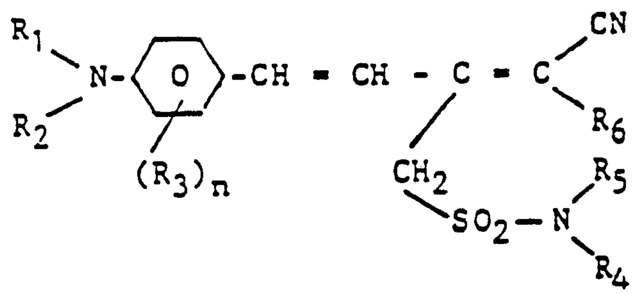

dans laquelle

$R_1$ et $R_2$ sont identiques ou différents, et représentent un atome d'hydrogène, un radical alkyle en $C_1$-$C_8$ ou phényle, éventuellement substitué une ou plusieurs fois par des atomes d'halogène ou par des groupes hydroxy, cyano ou alcoxy en $C_1$-$C_4$;

$R_3$ représente un atome d'hydrogène ou un groupe alkyle ou alcoxy en $C_1$-$C_4$;

n vaut de 1 à 4; ou

$R_1$, $R_2$ et $R_3$ forment conjointement avec le reste aminophényle correspondant un radical julolidinyle ou N-alkyl($C_1$-$C_3$)-carbazol-3-yle;

$R_4$ et $R_5$ représentent des atomes d'hydrogène ou les valences requises pour la formation d'un produit de condensation avec des dérivés du formamide, et

$R_6$ représente le groupe cyano, ou

$R_4$ représente un atome d'hydrogène et $R_5$ et $R_6$ forment ensemble le groupement

$$>C=N-R_7,$$

$R_7$étant un radical benzoyle substitué par des atomes d'halogène ou par des groupes alkyle ou alcoxy en $C_1$-$C_4$) cyano, amino ou mono- ou dialkyl($C_1$-$C_4$)-amino.

2. Dérivés de styrène selon la revendication 1, caractérisés en ce que

$R_1$ représente le groupe méthyle, éthyle ou ω-chloréthyle,

$R_2$ représente le groupe méthyle ou éthyle;

$R_3$ est un atome d'hydrogène ou le groupe méthoxy;

n est égal à 1, ou

$R_1$, $R_2$ et $R_3$ forment, conjointement avec le reste aminophényle correspondant, le radical julolidinyle;

$R_4$ et $R_5$ représentent des atomes d'hydrogène, et

$R_6$ représente le groupe cyano.

3. Dérivés de styrène selon la revendication 1, caractérisés en ce que $R_4$ et $R_5$ forment ensemble le groupe diméthylaminométhine.

4. Dérivés de styrène selon la revendication 1, caractérisés en ce que $R_4$ et $R_5$ forment ensemble le groupe 4,6-diméthylpyrimidin-2-yl-aminométhine.

5. Dérivés de styrène selon la revendication 1, caractérisés en ce que $R_4$ représente un atome d'hydrogène, et $R_5$ et $R_6$ forment ensemble le groupement

$$>C=N-R_7$$

et $R_7$ représente un radical benzoyle non substitué ou substitué par un groupe méthoxy ou cyano.

6. Procédé pour la préparation des styrènes sulfonés selon l'invention, de formule générale I selon la revendication 1, caractérisé en ce que l'on fait réagir des composés de formule générale VI

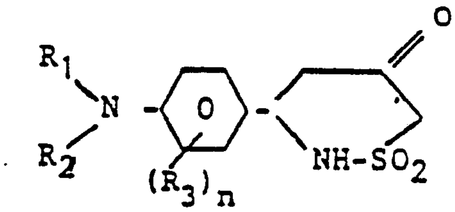

dans laquelle $R_1$, $R_2$, $R_3$ et n sont tels que définis dans la revendication 1, avec le malonitrile, dans un solvant inerte, à des températures dans la plage de 30 à 150° C, on isole par filtration les composés de formule générale II obtenus, et éventuellement on les purifie par recristallisation.

7. Procédé selon la revendication 6, caractérisé en ce que l'on fait réagir des composés de formule générale II avec des formamidacétals de formule VIII (équation de réaction 2) dans un solvant, à des températures dans la plage de 65 à 120° C, on isole le produit de réaction de formule III et on le purifie par recristallisation.

8. Procédé selon la revendication 6, caractérisé en ce que l'on fait réagir des composés de formule générale II avec des esters d'acide orthoformique de formule IX et une 2-amino-4,6-dialkylpyrimidine de formule X (équation de réaction 3) dans un solvant inerte, à des températures dans la plage de 60 à 140° C, on isole le produit de réaction et, éventuellement, on le purifie par recristallisation.

9. Procédé selon la revendication 6, caractérisé en ce que l'on fait réagir des composés de formule générale II avec des chlorures de benzoyle éventuellement substitués, de formule XI (équation de réaction 4), dans un solvant inerte, à des températures dans la plage de 30 à 100° C, on sépare les insolubles par filtration, on élimine le solvant par distillation et on purifie par recristallisation le produit de réaction obtenu.

10. Utilisation des dérivés de styrène sulfonés selon les revendications 1 à 5, en tant que colorants.

11. Utilisation des dérivés de styrène sulfonés selon les revendications 1 à 5, en tant que sensibilisateurs dans des matériaux de reproduction électrophotographique.

12. Utilisation des dérivés de styrène selon les revendications 1 et 11, pour la production de formes d'impression ou de circuits imprimés.